# EUROPEAN PATENT APPLICATION

(11) **EP 3 000 385 A1**
(43) Date of publication of application: **30.03.2016**
(21) Application number: 14003316.8
(22) Date of filing: 25.09.2014
(51) Int. Cl.: A61B 5/00, A61B 5/0408

(54) **Apparatus and method for measuring bio-potentials of a person**

(71) Applicant: University of Maribor, 2000 Maribor (SI)
(72) Inventor: Zazula, Damjan, 3320 Velenje (SI); Cigale, Boris, 2000 Maribor (SI); Kranjec, Jernej, SL-8280 Brestanica (SI); Kranjec, Primoz, 8280 Brestanica (SI); Sprager, Sebastijan, 2327 Race (SI)
(74) Representative: Rentsch Partner AG

(57) **Abstract**

An apparatus (100) for measuring one or more bio-potentials of a person is disclosed. The apparatus (100) comprises a supporting device (101) for the person and a measuring unit (102), which is configured to determine capacitances of individual electrodes of a set of capacitive electrodes (300) being attached to the supporting device (101). The measuring unit (102) is configured to use determined capacitances to select a subset of capacitive electrodes (301, 302) out of the set of capacitive electrodes (300). The measuring unit is configured to use the selected subset of capacitive electrodes (301, 302) for measuring the one or more bio-potentials of the person.

## Description

### FIELD OF THE INVENTION

The present invention relates to an apparatus and a method for measuring bio-potentials of a person.

### BACKGROUND ART

In order to observe vital functions of a person or of an individual, various types of sensors are used, for example surface electrodes attached to the skin or needle electrodes inserted into tissue of the person. In some environments of daily life of a person, such as in the office or in the living environment at home, unobtrusive observation of human vital functions is desired or required. For example, when a person is sitting or lying on a supporting device, namely on a furniture such as a chair, a bed, a couch, a vehicle seat, etc., it may be desirable that vital functions can be observed without the need to attach a surface electrode to the skin of the person or to insert a needle into the tissue of the person. Knowledge of vital functions of a person may be helpful to determine if a person requires any kind of immediate or postponed intervention, to be given either by medical experts, caregivers, social workers, etc. For example, personal health observations are automatically sent to a monitoring centre, accompanied by early warnings on possible acute situations. Experts in the centre may promptly decide whether the observed person needs urgent help, such as in the case of threatening cardiac infarction, apnea, etc., or in case he or she is just becoming restless and an early visit of a social worker would be beneficial, just to mention two extreme cases.

Unobtrusive observation of the vital functions of a person may be performed using a plurality of sensors embedded into supporting devices or furniture used in daily life, such as into seats, beds, etc. The sensors may be arranged in such a way that a direct or indirect contact with a person using the supporting device is established. It is desirable that human vital functions may be detected reliably, in particular in case of an indirect contact of the person with the sensors, in case contact changes with time because of movements of the person, and in case of a transitory contact.

DE102012009973 discloses a device for measuring bio-potentials of a person. A seat for the person includes a plurality of sensors embedded in the rear surface of the seat so that an electrocardiogram can be detected. A left hand grip and a right hand grip are arranged, which can be gripped by a person, wherein channels of an electrocardiogram can be derived. An evaluation unit is connected to the sensors and is adapted for determining the electrocardiogram.

DE102012002037 discloses a device for analysing state of a driver, e.g. the health. The device has sensors distributed on a backrest of a vehicle seat to capacitive measure vital signs. The sensors are connected to a signal-processing module to amplify and filter ECG (electrocardiogram) signals. The time-domain and frequency-domain of the HRV (heart rate variability) are analysed to display the stress state and activity of the nervous system of the driver.

EP1 627597 discloses a system for unobtrusively measuring bioelectric signals of an individual. The system includes multiple sensors, some of which constitute a capacitive sensor. The sensors are embedded into an object used to support the individual, such as a chair, bed, or the like. The sensors are preferably arranged in the form of an array, with particular ones of the sensors being selectable from the array for measuring bioelectric signals.

### DISCLOSURE OF THE INVENTION

It is an object of the present invention to provide an apparatus and a method for measuring bio-potentials of a person which do not have at least some of the disadvantages of the prior art. In particular, it is an object of the present invention to provide an apparatus and a method for measuring bio-potentials of a person reliably.

According to the present invention, these objects are achieved through the features of the independent claims. In addition, further advantageous embodiments follow from the dependent claims and the description.

According to the present invention, the above-mentioned objects are particularly achieved in that an apparatus for measuring bio-potentials of a person is provided, wherein the apparatus comprises a supporting device for the person, wherein the supporting device has attached a set of capacitive electrodes, and wherein the apparatus comprises a measuring unit being configured: to determine capacitances of individual electrodes of the set of capacitive electrodes; to use determined capacitances to select a subset of capacitive electrodes out of the set of capacitive electrodes; and to use the selected subset of capacitive electrodes for measuring bio-potentials of the person. The supporting device may be an object of daily life, such as a chair, couch, bed, etc. The set of capacitive electrodes may be attached to the supporting device to provide direct or indirect contact with the person. By selection of a subset of capacitive electrodes based on measured capacitances, bio-potentials of the person can be measured reliably.

In an embodiment, the measuring unit is further configured to select the subset of capacitive electrodes by comparing determined capacitances with a threshold and/or by evaluating a ratio between determined capacitances. In case the capacitance is larger than a threshold, the distance between the electrode and the person is small and measurement of bio-potentials is improved. In case the ratio between determined capacitances is close to one, the distance between the person and the electrodes is similar and measurement of bio-potentials is improved.

In an embodiment, the measuring unit is further configured to select the subset of capacitive electrodes by taking into account the inter-electrode distance between the capacitive electrodes. The inter-electrode distance may be stored in a corresponding table. In case the inter-electrode distance is large, the difference of bio-potentials measured by the electrodes is large as well.

In an embodiment, the measuring unit is further configured to select the subset of capacitive electrodes by selecting one or more pairs of capacitive electrodes having the highest capacitances and the largest inter-electrode distance. The high capacitance and large distance provides for both of a good contact and of large bio-potentials.

In an embodiment, the measuring unit and/or the capacitive electrodes are configured such that one or more capacitances are determined essentially at the same time as when measuring of one or more bio-potentials of the person occurs. Selection of appropriate electrodes can be adaptively changed during the measuring of bio-potentials.

In an embodiment, the measuring unit is further configured to estimate positional parameters of the person using determined capacitances. Accordingly, information about the person is enhanced.

In an embodiment, the measuring unit is further configured to determine the capacitance of individual electrodes by using a sample-and-hold capacitor. Capacitances are determined accurately using a robust technology.

In an embodiment, the measuring unit is further configured to select the subset of capacitive electrodes at one or more specific points in time. For example, after a specific time interval selection of the subset of electrodes can be adapted, wherein speed of adaptation can be controlled through appropriate definition of the time interval.

In an embodiment, the measuring unit is further configured to connect one or more electrodes to one out of one or more reference potentials. In particular, electrodes which are not used for measurement can be connected to a reference potential in order to improve signal quality of the bio-potentials.

In an embodiment, the measuring unit is further configured to measure bio-potentials with respect to a virtual common and to subtract the virtual common from the measured bio-potentials. By using a virtual common and by subtracting the virtual common, the dynamic range can be improved.

In addition to an apparatus for measuring bio-potentials of a person, the present invention further relates to a method of measuring bio-potentials of a person, the method comprising: determining capacitances of individual electrodes of a set of capacitive electrodes being attached to a supporting device for the person; using determined capacitances to select a subset of capacitive electrodes out of the set of electrodes; and using the selected subset of capacitive electrodes for measuring bio-potentials of the person.

In an embodiment, the method further comprises: selecting the subset of capacitive electrodes by comparing determined capacitances with a threshold and/or by evaluating a ratio between determined capacitances.

In an embodiment, the method further comprises: selecting the subset of capacitive electrodes by taking into account the inter-electrode distance between the capacitive electrodes.

In an embodiment, the method further comprises: selecting the subset of capacitive electrodes by selecting one or more pairs of capacitive electrodes having the highest capacitances and the largest inter-electrode distance.

Moreover, the invention relates to a computer program product comprising a non-transitory computer-readable medium having stored thereon computer program code for controlling one or more processors of an apparatus for measuring bio-potentials of a person such that the following steps are performed: determining capacitances of individual electrodes of a set of capacitive electrodes being attached to a supporting device for the person; using determined capacitances to select a subset of capacitive electrodes out of the set of capacitive electrodes; and using the selected subset of capacitive electrodes for measuring bio-potentials of the person.

A U-shaped electrode is disclosed, which is attached to a supporting device such as to the armrest of a chair, and which may be used to measure bio-potentials of a person. In a variant, the U-shaped electrode includes one or more additional sensors for simultaneous measurement of various vital signals of the person, such as bio-potentials, blood parameters, temperature, etc.

Using the described apparatus and method, the bio-potentials of an animal instead of the bio-potentials of a person or an individual may be measured equally well.

### BRIEF DESCRIPTION OF THE DRAWINGS

The herein described invention will be more fully understood from the detailed description given herein below and the accompanying drawings which should not be considered limiting to the invention described in the appended claims. The drawings are showing:
- Fig. 1: illustrates an embodiment of an apparatus for measuring one or more bio-potentials of a person, wherein a chair is configured to support the person and includes a set of capacitive electrodes in the backrest and the seat, and wherein a measuring unit is configured to use capacitive electrodes for measuring bio-potentials of the person sitting in the chair;
- Fig. 2: illustrates an embodiment of an apparatus for measuring one or more bio-potentials of a person, wherein a chair includes a set of capacitive electrodes in the backrest and the seat, one or more accelerometers, U-shaped sensors attached to armrests of the chair, and a measuring unit for measuring bio-potentials using capacitive electrodes and for controlling and/or processing signals of the one or more accelerometers and the U-shaped sensors;
- Fig. 3: illustrates an embodiment of an U-shaped sensor, comprising electrodes, photoplethysmographic light sources and optical sensors, and temperature sensors;

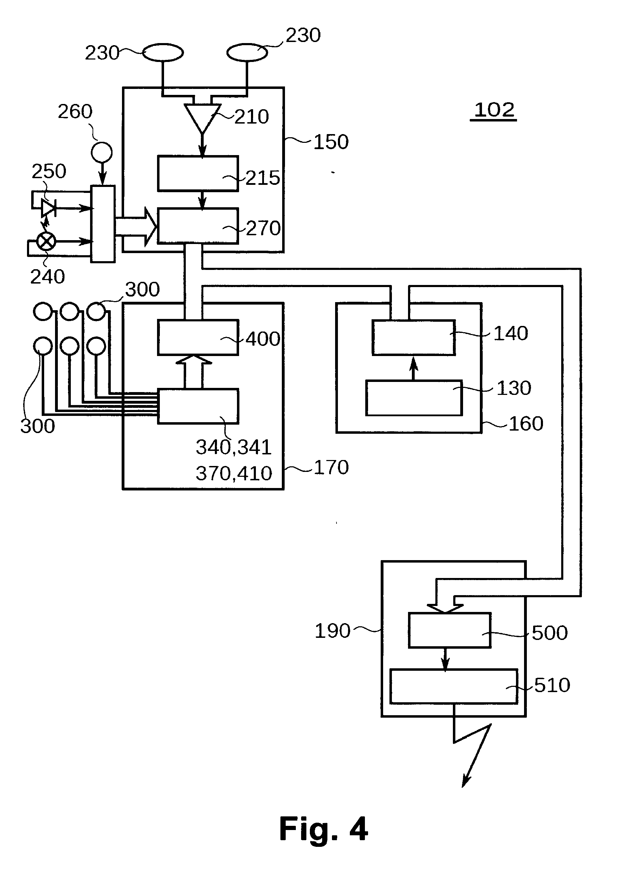

- Fig. 4: illustrates a block diagram of a measuring unit for measuring one or more bio-potentials of a person, wherein capacitive electrodes are connected to a unit for selecting capacitive electrodes and for measuring bio-potentials of a person using the selected capacitive electrodes, and wherein U-shaped sensors comprising electrodes, photoplethysmographic light sources and optical sensors, and temperature sensors are connected to a unit for controlling and/or processing signals of the U-shaped sensors comprising electrodes, photoplethysmographic light sources and optical sensors, and temperature sensors;
- Fig. 5: illustrates a block diagram of a unit for selecting capacitive electrodes and for measuring bio-potentials of a person using the selected capacitive electrodes;
- Fig. 6: illustrates a capacitive electrode comprising an electrode surface for determining a capacitance of the electrode, an electrode surface for measuring bio-potentials, and a guard;
- Fig. 7: is a flow diagram illustrating aspects of operations that may be performed for measuring one or more bio-potentials of a person;
- Fig. 8: is a flow diagram illustrating aspects of operations that may be performed for measuring one or more bio-potentials of a person, wherein capacitances are compared to a threshold, and wherein a capacitive ratio is compared to a threshold;
- Fig. 9: is a flow diagram illustrating aspects of operations that may be performed to determine capacitances of individual electrodes;
- Fig. 10: is a flow diagram illustrating aspects of operations that may be performed to select from a set of electrodes the best pair of electrodes for measuring bio-potentials of a person; and
- Fig. 11.1, 11.2: is a flow diagram illustrating aspects of operations that may be performed to simultaneously determine capacitances of individual electrodes and measure bio-potentials.

### MODE(S) FOR CARRYING OUT THE INVENTION

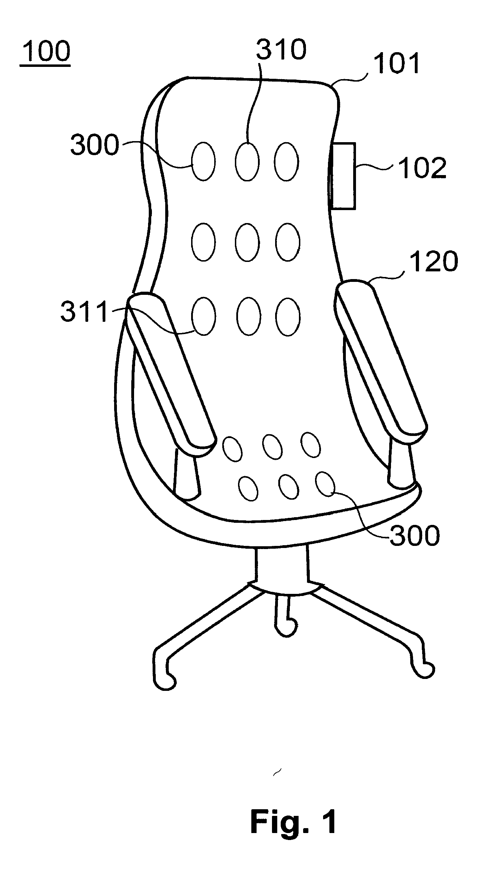

Figure 1 shows an apparatus 100 for measuring one or more bio-potentials of a person. The apparatus 100 includes a supporting device for the person, namely a chair 101, and a measuring unit 102. The supporting device is configured to support the person in a comfortable manner. The supporting device may include furniture such as a chair, a bed, a couch, a vehicle seat, etc. Parts of the measuring unit 102, such as a control interface, a graphical user interface, etc., may be included in remote devices, e.g. in a remote desktop personal computer, a notebook computer, a tablet computer, a smartphone, etc. The measuring unit 102 includes electronic circuitry comprising amplifiers, multiplexers, computer processors, etc. in order to perform the necessary functions and operations as described above and in the following. These functions and operations may be provided using embodiments in software, in hardware, or any combination thereof.

Sensors are attached to the supporting device, namely to an object of daily life, such as a chair 101, a couch, etc. The sensors measure vital functions of the person using sensors attached to the supporting device or object of daily life. In a variant, the sensors measure bio-potentials through direct or indirect contact with the palms, fingers, back, backside, arms, legs, etc. In a variant, the sensors measure mechanical and/or acoustic vibrations due to the activity of internal organs, such as the heart, lungs, stomach, etc. In a variant, the sensors measure mechanical movements of the person, such as movements of the body, body parts, etc. In a variant, the sensors measure absolute or relative values, changes, etc. of pulsatile pressure in blood vessels, of blood oxygenation, of the temperature of body parts, etc.

In the embodiment shown in Fig. 1, the backrest and the seat of the chair 101 comprise a set of capacitive electrodes 300. In a variant, only the backrest or only the seat comprise a set of capacitive electrodes 300. Any number of electrodes may be arranged in the chair 101. In a variant, the electrodes 300 are arranged according to a regular or irregular grid.

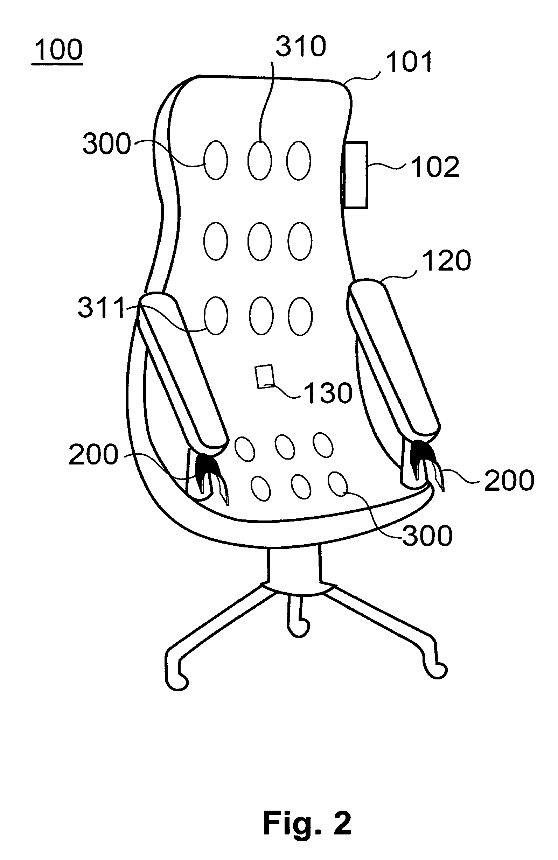

Figure 2 shows another embodiment of an apparatus 100 for measuring bio-potentials of a person. The apparatus 100 includes a supporting device for the person, namely a chair 101, and a measuring unit 102. In a variant, parts of the measuring unit 102, such as a control interface, a graphical user interface, etc., are included in remote devices, e.g. in a remote desktop personal computer, a notebook computer, a tablet computer, a smartphone, etc. The chair 101 includes a set of capacitive electrodes 300, U-shaped sensors 200 arranged at the armrests 120, and one or more inertial measurement elements 130, e.g. accelerometers. The U-shaped sensors 200 are arranged at the end of the armrests 120 and are configured that a finger of a person having an arm put on the armrest 120 easily establishes contact with the U-shaped sensors 200. In a variant, the U-shaped sensors 200 include electrodes 230, e.g. dry electrodes designed to be in direct contact with specific parts of the skin of a person, e.g. the skin of a finger. The one or more inertial measurement elements 130 are configured to detect any movement of the chair, such as movements of the chair 101 because a person sitting on the chair 101 is moving the chair around.

In a variant, the measuring unit 102 shown in Fig. 1 and Fig. 2 is configured to determine proximity of a person sitting on the chair 101, respectively using the supporting device or object of daily life, by measuring capacitances using the capacitive electrodes 300, in particular by measuring capacitances between the body of the person and the capacitive electrodes 300. Using the measured capacitances, the person's posture, movements while being seated, etc. may be estimated and used to provide ergonomic parameters about the person sitting on the chair 101. At the same time, the capacitive electrodes 300 are used to measure bio-potentials of the person, as described above and below, wherein the capacitive electrodes 300 are used to perform an adaptive measurement of bio-potentials, in particular by adaptively selecting appropriate capacitive electrodes 300 used for measuring one or more bio-potentials of the person, which improves signal quality and which increases signal-to-noise ratio.

In typical applications, one or more U-shaped sensors 200 are arranged on supporting devices such as chairs, benches, sofas, wheelchairs, etc. in the office or living environment of a person. Accordingly, when a person uses a supporting device, e.g. sits on a chair 101 according to Fig. 2 and puts the arms on the armrests 120, fingers of the person slide easily into the U-shaped sensors 200. This is in particular because of the location, shape, size, geometry, etc. of the U-shaped sensors 200 being adapted to receive a finger of a person, wherein a firm contact is established between the finger and the U-shaped sensors 200. Accordingly, when U-shaped sensors 200 are arranged at the lower side of the armrest 120 of a chair 101, a good contact between the fingers of a person and the U-shaped sensors 200 is established. In a variant, an electrode 230 of an U-shaped sensor 200 has a dry surface, which make possible a direct contact to the fingers of the person and therefore a dry measurement of bio-potentials is enabled, in particular for the measurement of the electrocardiogram (ECG). In a variant, the U-shaped sensor 200 is elastic, wherein a specific pressure is established between the U-shaped sensor 200 and a finger of a person arranged in the U-shaped sensor 200 in order to provide for a good contact between the U-shaped sensor 200, e.g. an electrode or another sensor, and the finger.

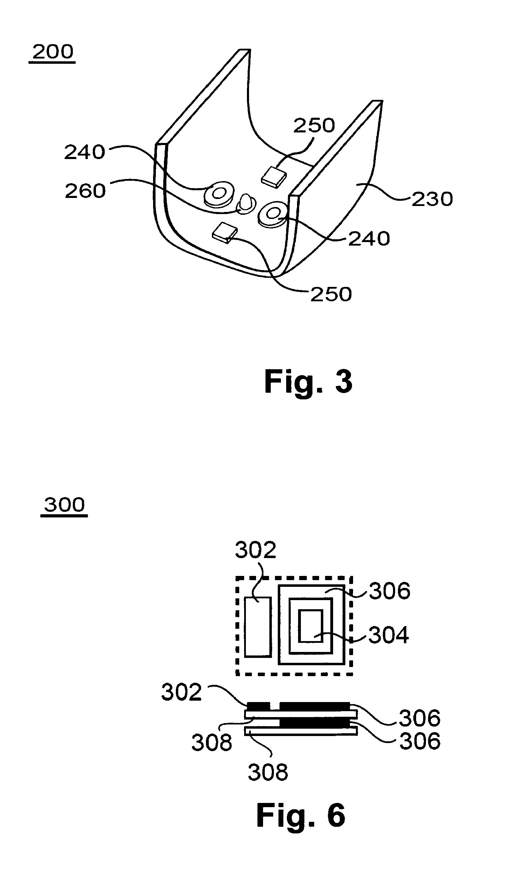

Figure 3 illustrates an embodiment of a U-shaped sensor 200 including an electrode 230 configured to measure bio-potentials such as the electrocardiogram (ECG). The electrode 230 of the U-shaped sensor 200 may include several parts. The electrode 230 may be made of a conductive material, such as copper, steel, aluminium, etc. In a variant, the U-shaped sensor 200 includes one or more photoplethysmographic light sources 240 and one or more optical sensors 250. Accordingly, the U-shaped sensor 200 is configured to acquire photoplethysmographic signals (PPG) in order to observe heartbeat, blood oxygenation, blood density, blood vessel compliance, etc. of a person. In a variant, photoplethysmographic light sources 240 and/or optical sensors 250 operate at different wavelengths. In a variant, by combining the measured PPG and ECG, blood pressure can be determined. In a variant, the U-shaped sensor 200 includes one or more temperature sensors 260, e.g. thermistors, etc. Accordingly, the U-shaped sensor 200 is configured to measure the temperature of a person. The signals acquired by the U-shaped sensors 200 are synchronized in time, which enables synchronized processing of the signals and estimation of additional vital parameters such as the blood pressure.

Figure 4 illustrates an measuring unit 102 for measuring bio-potentials of a person and for processing further signals. Capacitive electrodes 300 are connected to a unit 170 to select capacitive electrodes and to use the selected electrodes for measuring bio-potentials of a person. U-shaped sensors 200, which include electrodes 230, photoplethysmographic light sources 240 and optical sensors 250, and temperature sensors 260, are connected to a unit 150 for controlling and/or processing signals of the U-shaped sensors 200, namely of the electrodes 230, photoplethysmographic light sources 240 and optical sensors 250, and temperature sensors 260.

As illustrated in Fig. 4, the unit 170, which is configured to select capacitive electrodes and to use the selected electrodes for measuring bio-potentials of a person, includes a processing unit 400 and multiplexers 340, 341, 370, 410. In a variant, processing unit 400 includes a microcontroller.

As illustrated in Fig. 4, signals from the electrodes 230 of the U-shaped sensors 200 are differentially amplified by amplifier 210 and notch filtered at mains frequency (50 Hz, 60 Hz) by filters 21 5. In a variant, amplifier 210 is a ultra-high input impedance amplifier needed for capacitive measurements of bio-potentials. In a variant, the filters 215 are analogue filters performing a low-pass and a high-pass filtering operation as well, for example having cut-off frequencies at 100 Hz and 0.5 Hz respectively. In a variant, the electrode 230, the one or more photoplethysmographic light sources 240 and the one or more optical sensors 250 of the U-shaped sensor 200 are connected to a processing unit 270, which includes a sampling device and an analogue-to-digital converter to digitise acquired ECG and PPG signals. In case both the ECG and PPG signals are acquired, the signals are fully synchronized in time, which enables estimation of the blood pressure based on a comparison of signal features. In a variant, processing unit 270 is adapted to acquire signals of the one or more temperature sensors 260. In a variant, processing unit 270 includes a microcontroller.

As illustrated in Fig. 4, one or more inertial measurement elements 130 are connected to a processing unit 140. In a variant, the one or more inertial measurement elements 130 are included in inertial unit 160. The processing unit 140 is configured to acquire signals of the one or more inertial measurement elements 130 in order to estimate or determine motion of parts of an supporting device, such as the backrest of a chair 101, or an entire supporting device, such as a chair 101. In a variant, the trajectory and/or the intensity of estimated or determined motion are used to estimate or determine activity of the person and/or its vital functions. In a variant, processing unit 140 includes a microcontroller.

As illustrated in Fig. 4, a processing unit 500 is configured to collect data and measurements from units 150, 160, 170. All data and measurements are synchronized in time which aligns all different observations of vital functions and enables further analysis of the vital functions, for example the estimation of blood pressure. As illustrated in Fig. 4, a transmitter 510 is configured to transmit acquired data, measurements, and/or analysis results to a computer network. In a variant, transmitter 510 is configured to transmit data to a desktop personal computer, to a notebook computer, to a tablet computer, to a smartphone, etc. connected to the computer network. In a variant, processing unit 500 and transmitter 510 are arranged in transmission unit 190. In a variant, processing unit 500 includes a microcontroller.

Figure 6 illustrates a capacitive electrode 300 comprising an electrode surface 302 for determining a capacitance of the electrode, an electrode surface 304 for measuring bio-potentials, and a guard 306. Accordingly, each capacitive sensor 300 comprises three separated conductive surfaces. As illustrated in Fig. 6, surface 304 for measuring bio-potentials is surrounded at the same level by another surface 306 forming the guard. However, the guard 306 also extends beneath the measuring surface 304, so that it actually encapsulates, i.e. protects, the measuring surface in space (around and below). In a variant, a two-sided printed board is etched such that the guard 306 is formed on the top (cf. upper ground plan in Fig. 6) and also beneath, both wired through the two-sided printed board. As illustrated in Fig. 6, the guard is wrapped around the surface 304 for measuring bio-potentials. In Fig. 6, the conductive layers are filled with black, whereas empty rectangles designate a non-conductive support 308 for the conductive parts, the non-conductive support 308 keeping together the conductive parts. Accordingly, the capacitive electrode 300 comprises three separated conductive surfaces and two non-conductive supports to carry the layers, as depicted in Fig. 6.

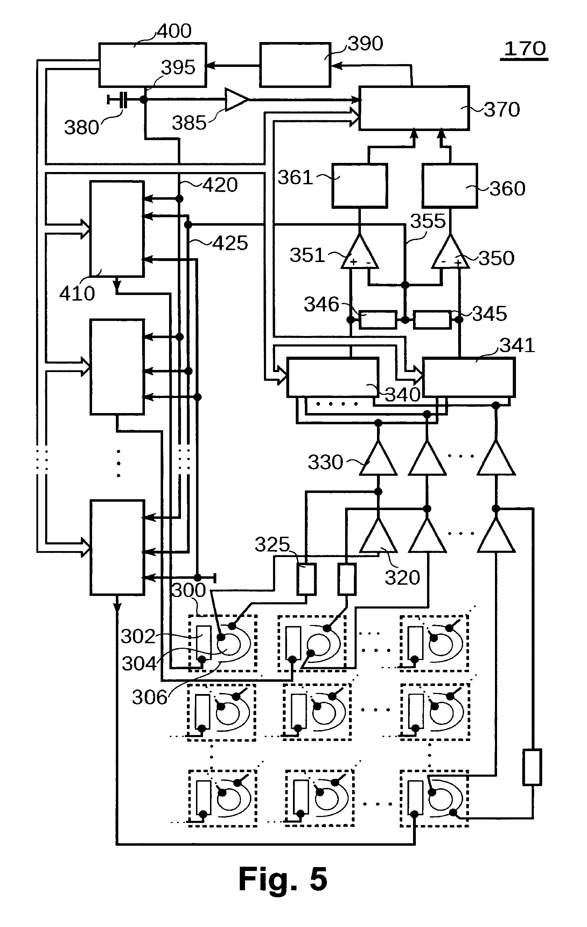

Figure 5 illustrates a block diagram of a unit 170 for selecting capacitive electrodes of a supporting device for a person and to use the selected electrodes for measuring bio-potentials of the person using the supporting device. As illustrated in Fig. 5, a plurality of (disjunctive) capacitive electrodes 300 is arranged in a grid and forms a set of capacitive electrodes 300. Electrodes may be mounted in the inner or outer side of the supporting device, e.g. a chair 101, namely the backrest or the seat of a chair 101, that the person is using and is having in direct or indirect contact with the body or a body part. When the person using the supporting device is moving, e.g. is sitting and moving on the chair 101, the capacitances of the electrodes 300 vary. The measured capacitances reflect the type and intensity of the movements. At the same time, the capacitive sensors 300 measure bio-potentials of the person in a capacitive manner, namely bioelectric signals of the person are measured. In a variant, at least one pair of capacitive sensors 300 is involved in the acquisition of differential signals. In a variant, the remaining electrodes are connected to the ground or to virtual common 355. The optimal distribution of potentials over the electrodes 300 is assessed using a procedure which takes into account the capacitances of the electrodes 300, wherein a switching circuit (comprising multiplexers 410 controlled by processing unit 400, e.g. a microcontroller) drives every capacitive electrode 300 of the set of electrodes (arranged in a grid, for example) in order to improve common-mode rejection ratio (CMRR). In a variant, each capacitive electrode 300 is connected to a corresponding multiplexer 41 0, which may be separate from other multiplexers. Accordingly, capacitive electrodes 300 can be connected to three different potentials: to the potential of the sample-and-hold capacitor 380, to the ground potential, or to the potential of virtual common 355. In a variant, virtual common 355 is obtained based on the measurement of bio-potentials on a pair of selected capacitive electrodes 300. The electrode surface 304 for measuring bio-potentials of each electrode 300 is connected to a voltage follower 330 which drives corresponding inputs of two analogue multiplexers 340, 341. The addresses of the electrodes from a selected pair are latched one in the multiplexer 340 and the other in multiplexer 341. In this way, the potentials from the two electrodes appear at the outputs of the two multiplexers 340, 341. Through resistors 345, 346, the two potentials sump up into a virtual common 355. Virtual common 355 is implemented in further differential amplification of the potentials of selected electrodes. Op-amps 350, 351 provide for this functionality. The amplified potentials are further processed by the filters 360, 361. In a variant, the filters 360, 361 provide an extremely strong suppression of mains frequency (50Hz, 60Hz). The filtered potentials are selected and passed to analogue-to-digital converter 390 using a further analogue multiplexer 370. In a variant, the bit resolution of analogue-to-digital converter 390 is large, for example 24bits or larger, and adapted to the large dynamic range of bio-potential measurements. In spite of good CMRR and adaptable robustness of the introduced circuitry, the amplitudes of the bio-potential measurements may be very low.

As illustrated in Fig. 5, a sample-and-hold capacitor 380 is used for measuring the capacitance of the capacitive electrodes 300. The sample-and-hold capacitor 380 is charged first. In a next step, the sample-and-hold capacitor 380 is switched over for a predetermined time interval to one of the capacitive electrodes 300, in particular using multiplexer 410. In a next step, after the time interval, the remaining voltage and/or charge of the sample-and-hold capacitor 380 is determined, which is inversely proportional to the capacitance of the capacitive electrode 300 under investigation. The electronic circuits of the unit 170 (cf. Fig. 4) are multiplexed, so that they alternately sample the capacitance of capacitive electrodes 300 and the bio-potentials acquired by the capacitive electrodes 300. Using the sampled capacitance of the capacitive electrodes 300, rules are applied in order to determine capacitive electrodes 300 used for bio-potential measurements and unused capacitive electrodes 300, which are connected either to potential of ground or virtual common 355 for suppressing interferences, such as mains (50Hz, 60Hz). Adaptive wiring is supported by a special wiring circuitry in the unit 170, comprising multiplexers 340, 341, 370, 41 0, as illustrated in Fig. 5.

In a variant, photoplethysmographic (PPG) circuitry from Fig. 4 implements a known solution for pulse oximetry, in particular by illuminating the blood with two light sources of different wavelengths. A built-in microcontroller turns alternately on and off the light-emitting diodes 240 (LEDs) of red and infrared wavelengths. At the same time, it acquires a response from at least one optical sensor 250 whose embodiment is preferably based on an array sensor with in-line photodiodes.

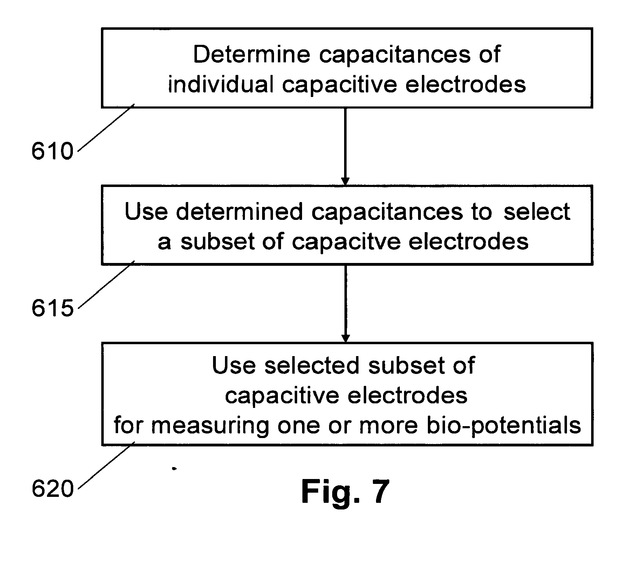

Figure 7 is a flow diagram illustrating aspects of operations that may be performed for measuring one or more bio-potentials of a person. In step 610, capacitances of individual capacitive electrodes 300 attached to a supporting device, e.g. a chair 101, are determined. In step 61 5, the determined capacitances are used to select a subset of capacitive electrodes. In step 620, the selected subset of capacitive electrodes is used to measure one or more bio-potentials of a person using the supporting device, e.g. the chair 101.

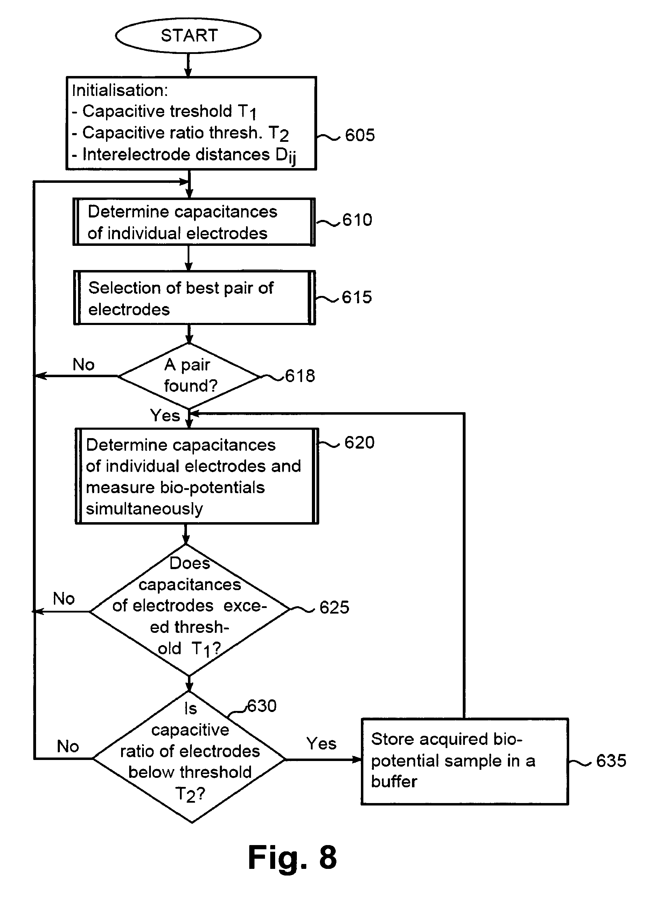

Figure 8 is a flow diagram illustrating aspects of operations that may be performed for measuring one or more bio-potentials of a person, wherein capacitances are compared to a threshold, and wherein a capacitive ratio is compared to a threshold. In step 605, the constants are initialized: two thresholds, T1 and T2, and inter-electrode distances Dij of the capacitive electrodes 300. Threshold T1 is empirical and defines the minimum electrode 300 capacitance that still keeps bio-potential measurements feasible. Also Threshold T2 is empirical and defines a ratio of the electrode capacitances for a pair of electrodes selected for bio-potential measurements. If this ratio equals 1, then the measured bio-potential equals exact difference between the voltages of the two electrodes involved. When the ratio differs from 1, the measured bio-potential approaches to one of the electrode voltages only. This affects the measured signal form and is undesirable. Therefore, only measurements with capacitances above T1 and their ratio close to 1 are accepted.

As illustrated in Figure 8, in step 610 procedure to determine capacitances of all the electrodes 300 is performed. This is done at the beginning and occasionally, when the selected pair of electrodes cannot provide bio-potential measurements of a sufficient quality any more. In a variant, at the same time, information on the capacitances of all the electrodes is involved in an assessment of subject's body proximity to the electrodes: the higher the electrode capacitance the closer is this distance. A set of electrodes 300 thus enables conclusions on the body posture referring to capacitances of the set of electrodes.

As illustrated in Figure 8, in step 615, when all electrode capacitances are known, a method for selecting the best pair is initiated. It is possible that no suitable pair can be found. For example, if the person is too far from the capacitive electrodes 300, body-to-electrode coupling is too weak to measure bio-potentials. If this happens, a "No signal" indicator is on and in step 618 a comparison branches to a new call to the measurement of all the electrode capacitances, i.e. step 610 is performed.

As illustrated in Figure 8, if detection of bio-potentials is feasible, in step 620 simultaneous acquisition of the electrode capacitances and the bio-potentials on the pair of selected electrodes commences. After every sample taken, the suitability of the selected electrode pair is tested. Firstly, the electrode capacitances must exceed threshold T1 (in accordance to decision 625), and secondly, the ratio of the electrode capacitances must be within the threshold T2 tolerances, i.e. close to 1 (in accordance to decision 630). As long as the conditions are fulfilled, in step 635 the acquired bio-potential samples or measurements as stored in a buffer for further usage. Once the condition according to one of decision 625 or 630 fails, a new best pair of electrodes ought to be looked for, which calls for another measurement of capacitances of all the electrodes, i.e. step 610 is performed.

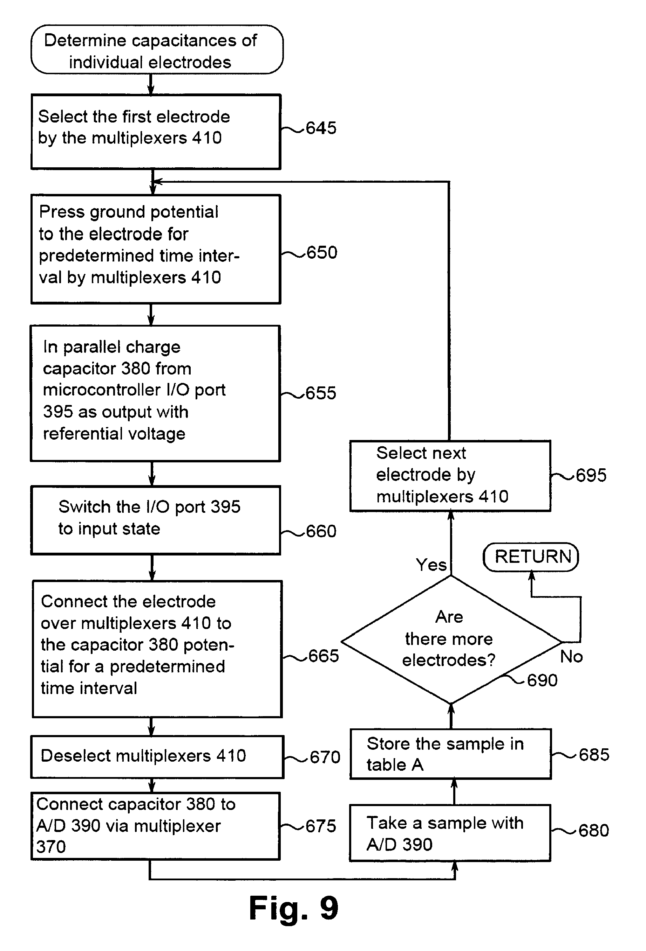

Figure 9 is a flow diagram illustrating aspects of operations that may be performed to determine capacitances of individual electrodes. In step 645, the first electrode in the set is addressed using the multiplexer 410 that is responsible for this electrode in the way that ground potential is attached to the electrode for a predetermined time interval in step 650. This step is necessary to guarantee that no charge is residing on the electrode before its capacitance is being measured. In the meantime, in step 655, sample-and-hold capacitor 380 is charged by a referential voltage conveyed from an I/O port 395 that is pushed into its output state by the microcontroller 400. After a predetermined time interval, in step 660 the I/O port 395 is switched to its input state, meaning that it flips into high-impedance state and, in fact, disconnects from the capacitor 380. Now, in step 665, the selected electrode's multiplexer 410 promptly connects capacitor 380 to the selected electrode 300. After a predetermined time interval, a certain amount of the charge flows from the capacitor to the electrode. The amount depends on the proportion of their capacitances. The higher the capacitance of the electrode, the more significant is the capacitor 380 discharge and, consequently, a drop of voltage on it. In step 670, the electrode is then disconnected from the capacitor 380, which is achieved by a deselection of multiplexer 410. In step 675, voltage on the capacitor 380 is sampled by the analogue-to-digital converter 390 when the two are interconnected through multiplexer 370. In step 680, a sample is acquired and stored in a table, e.g. in table A in step 685. In step 690, until all the electrodes are addressed, a selection of a next electrode is activated by the corresponding multiplexer 410 in step 695.

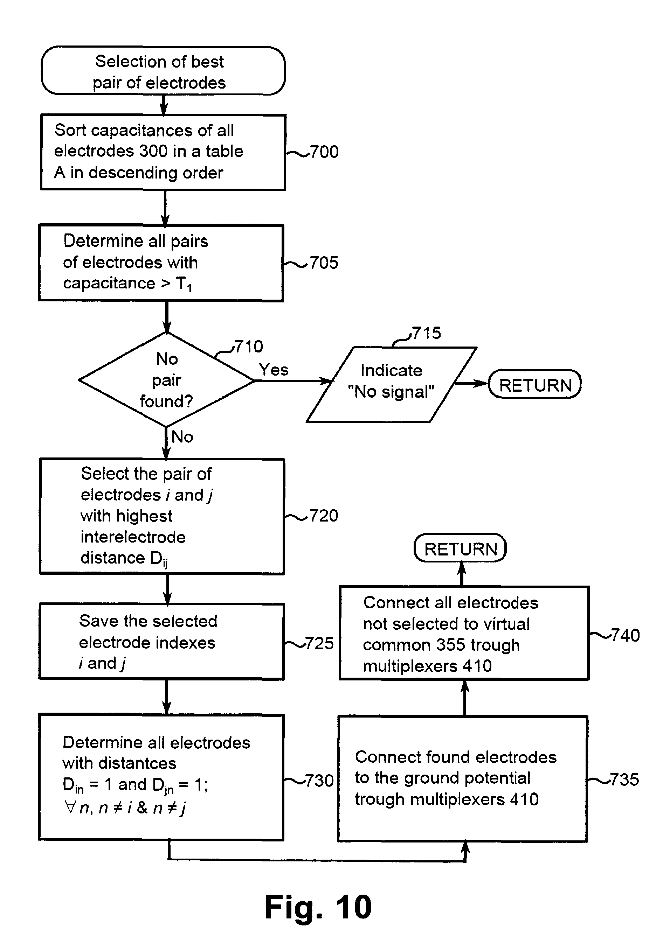

Figure 10 is a flow diagram illustrating aspects of operations that may be performed to select from a set of electrodes the best pair of electrodes for measuring bio-potentials of a person. The goal is to find such two electrodes whose properties warrantee acceptable bio-potential measurements. Measured capacitances of all the electrodes 300 have been saved in table A (cf. Fig. 9, step 685). In step 700, they are sorted first in descending order. In step 705, all electrodes whose capacitances exceed threshold T1 are compared in all possible pairs. If no such pair is found (in decision 71 0), a "No signal" indication is triggered in step 715. In step 720, when there are pairs of suitable electrodes available, those i-th and j-th electrodes are chosen that maximise their inter-electrode distance Dij. In step 725, selected electrode indexes i and j are saved. Now, the two selected electrodes destined for bio-potential measurement are encompassed by the potentials that diminish the measurement noises and improve signal's quality and signal-to-noise ratio. Empirical findings suggest ground potentials close to the measurement electrodes and virtual common potential in the middle between the measurement electrodes. Therefore, in step 730, electrodes with spatial distance of 1 from the electrodes i and j are looked for: Din=1 and Djn=1; ∀n, n≠i & n≠j. In step 735, these electrodes closest to the measuring electrodes i and j are connected to the ground potential, each by its corresponding multiplexer 410. In step 740, all remaining electrodes not allocated yet are connected to virtual common 355, again through their multiplexers 410.

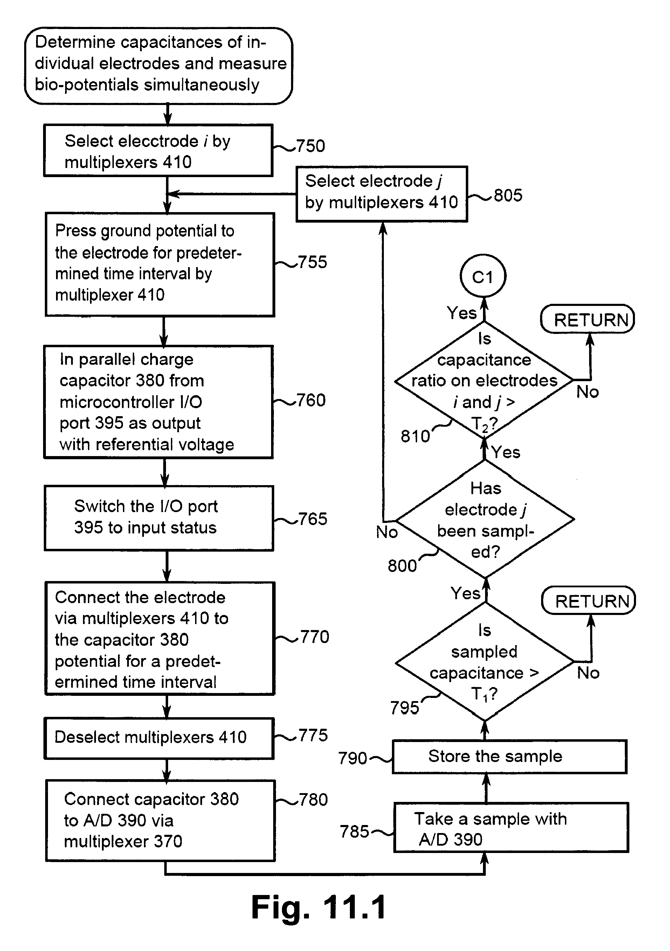

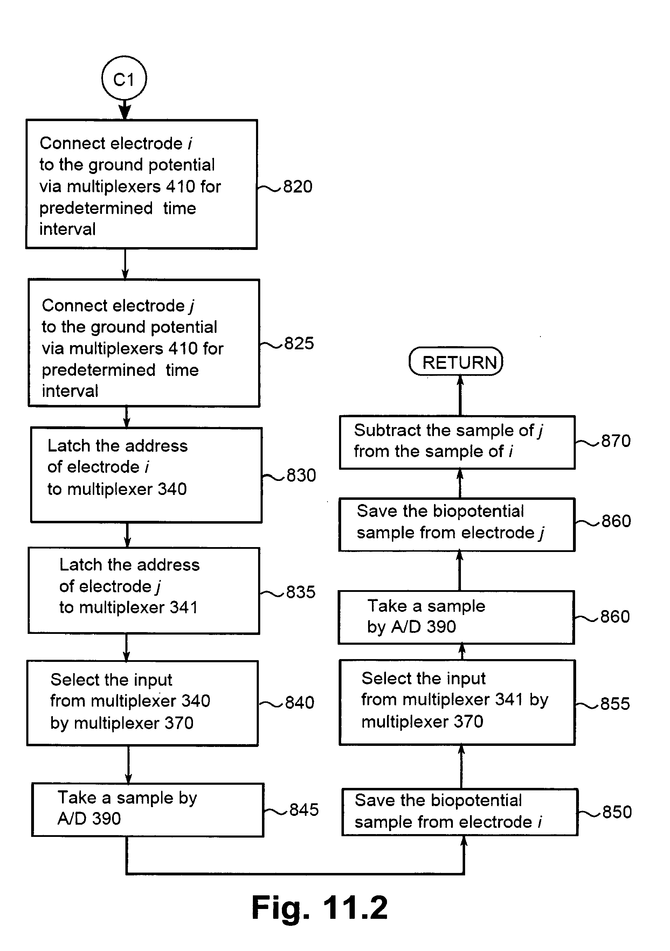

The combination of Figure 11.1 and Figure 11.2 is a flow diagram illustrating aspects of operations that may be performed to simultaneously determine capacitances of individual electrodes and measure bio-potentials. The aim is to manage the acquisitions and, in parallel, test whether the selected pair of measuring electrodes still fulfils criteria for bio-potential measurements having required quality. Therefore, in steps 750 to 785, the same steps are first implemented as in establishing capacitances of all the electrodes. Steps 750 to 785 serve the same purpose as steps 645 to 680 in Fig. 9. When the capacitance sample is saved for electrode i in step 790, its value is compared in step 795 to threshold T1. If the value exceeds the threshold, in step 800 and step 805, capacitance measurement continues with electrode j, otherwise current pair of measuring electrodes is discarded and a search for a new pair is initiated. After this measurement is completed in step 795 again, capacitances of the two measuring electrodes, i.e. the i-th and j-th one, are compared. If their ratio meets the threshold T2, acquisition of bio-potentials begins, otherwise, in step 810, current pair of measuring electrodes is discarded and a search for a new pair is initiated.

Bio-potentials are acquired as depicted in Fig. 11.2. Both measuring electrodes, i.e. the i-th and j-th, are first discharged in step 820 and step 825 by a connection to the ground potential via their multiplexers 410 for a predetermined time interval. This is necessary to avoid any induction of voltages from the stage of the electrode capacitance sampling. After that, in step 830 and step 835, the addresses of the two electrodes are latched into the multiplexers 340 and 341. The outputs of these multiplexers are wired to multiplexer 370. In step 840, multiplexer 370 selects the output voltage of multiplexer 340, i.e. the bio-potential on electrode i. Then, in step 845, the analogue-to-digital conversion takes places by the converter 390, and the acquired sample is stored in step 850. The same steps repeat for the electrode j in step 855, step 860, and step 865. Finally, in step 870, samples of voltages from electrodes i and j, that are measured against virtual common potential 355, are subtracted to obtain bio-potential measured between the two selected electrodes. This sample is stored to a buffer, according to step 635 of Fig. 8, to compose measured bio-potential signal.

## Claims

1. An apparatus (100) for measuring bio-potentials of a person, wherein the apparatus (100) comprises a supporting device (101) for the person, wherein the supporting device (101) has attached a set of capacitive electrodes (300), and wherein the apparatus comprises a measuring unit (102) being configured:
to determine capacitances of individual electrodes of the set of capacitive electrodes (300);
to use determined capacitances to select a subset of capacitive electrodes (301, 302) out of the set of capacitive electrodes (300); and
to use the selected subset of capacitive electrodes (301, 302) for measuring bio-potentials of the person.

2. The apparatus (100) according to claim 1, wherein the measuring unit (102) is further configured to select the subset of capacitive electrodes (301, 302) by comparing determined capacitances with a threshold (T1) and/or by evaluating a ratio between determined capacitances.

3. The apparatus (100) according to claim 1 or 2, wherein the measuring unit (102) is further configured to select the subset of capacitive electrodes (301, 302) by taking into account the inter-electrode distance between the capacitive electrodes (300).

4. The apparatus (100) according to one of claims 1 to 3, wherein the measuring unit (102) is further configured to select the subset of capacitive electrodes (301, 302) by selecting one or more pairs of capacitive electrodes (300) having the highest capacitances and the largest inter-electrode distance.

5. The apparatus (100) according to one of claims 1 to 4, wherein the measuring unit (102) and/or the capacitive electrodes (300) are configured such that one or more capacitances are determined essentially at the same time as when measuring of one or more bio-potentials of the person occurs.

6. The apparatus (100) according to one of claims 1 to 5, wherein the measuring unit (102) is further configured to estimate positional parameters of the person using determined capacitances.

7. The apparatus (100) according to one of claims 1 to 6, wherein the measuring unit (102) is further configured to determine the capacitance of individual electrodes by using a sample-and-hold capacitor.

8. The apparatus (100) according to one of claims 1 to 7, wherein the measuring unit (102) is further configured to select the subset of electrodes at one or more specific points in time.

9. The apparatus (100) according to one of claims 1 to 8, wherein the measuring unit (102) is further configured to connect one or more capacitive electrodes (300) to one out of one or more reference potentials.

10. The apparatus (100) according to one of claims 1 to 9, wherein the measuring unit (102) is further configured to measure bio-potentials with respect to a virtual common and to subtract the virtual common from the measured bio-potentials.

11. A method for measuring bio-potentials of a person, comprising:
determining (610) capacitances of individual electrodes of a set of capacitive electrodes (300) being attached to a supporting device (101) for the person;
using (615) determined capacitances to select a subset of capacitive electrodes (301, 302) out of the set of electrodes (300); and
using (620) the selected subset of electrodes (301, 302) for measuring bio-potentials of the person.

12. The method according to claim 11, further comprising: selecting the subset of capacitive electrodes (301, 302) by comparing determined capacitances with a threshold (T1) and/or by evaluating a ratio between determined capacitances.

13. The method according to claim 11 or 1 2, further comprising: selecting the subset of capacitive electrodes (301, 302) by taking into account the inter-electrode distance between the capacitive electrodes (300).

14. The method according to one of claims 11 to 13, further comprising: selecting the subset of capacitive electrodes (301, 302) by selecting one or more pairs of electrodes having the highest capacitances and the largest inter-electrode distance.

15. A computer program product comprising a non-transitory computer-readable medium having stored thereon computer program code for controlling one or more processors of an apparatus (100) for measuring bio-potentials of a person such that the following steps are performed:
determining (610) capacitances of individual electrodes of a set of capacitive electrodes (300) being attached to a supporting device (101) for the person;
using (615) determined capacitances to select a subset of capacitive electrodes (301, 302) out of the set of capacitive electrodes (300); and
using (620) the selected subset of capacitive electrodes (301, 302) for measuring bio-potentials of the person.
